Europäisches Patentamt

(19) European Patent Office

Office européen des brevets

(11) Publication number: **0 115 442**
A2

# (12) EUROPEAN PATENT APPLICATION

(21) Application number: 84300570.3

(22) Date of filing: 30.01.84

(51) Int. Cl.³: **A 61 K 39/245**
A 61 K 37/02, A 61 K 39/12

(30) Priority: 01.02.83 US 462841

(43) Date of publication of application:
08.08.84 Bulletin 84/32

(84) Designated Contracting States:
BE CH DE FR GB IT LI NL SE

(71) Applicant: THE UPJOHN COMPANY
301 Henrietta Street
Kalamazoo, Michigan 49001(US)

(72) Inventor: Rea, Thomas James
c/o The Upjohn Company 301 Henrietta Street
Kalamazoo Michigan 49001(US)

(72) Inventor: Brideau, Roger Joseph
c/o The Upjohn Company 301 Henrietta Street
Kalamazoo Michigan 49001(US)

(72) Inventor: Hamdy, Aziz Hamed
c/o The Upjohn Company 301 Henrietta Street
Kalamazoo Michigan 49001(US)

(72) Inventor: Post, Leonard Edwin
c/o The Upjohn Company 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) Vaccine and its use against pseudorabies virus.

(57) A vaccine comprising glycoprotein gX (also known as 3a) can be used to protect a PRV-susceptible animal, particularly swine, from PRV.

EP 0 115 442 A2

Croydon Printing Company Ltd.

VACCINE AND ITS USE AGAINST PSEUDORABIES VIRUS

This invention relates to a vaccine and its use against Pseudorabies virus.

Pseudorabies virus (PRV) is a herpesvirus which can infect most domestic animals to cause clinical symptoms ranging from vomiting, diarrhea, fever, respiratory and neurological disorders, and abortion, culminating, generally, in death to the host animal.

Pseudorabies is known as Aujeszky's disease, mad itch and bulbar paralysis. When PRV infects pigs, serious economic loss is incurred by the farmer. Very young pigs die whereas older pigs get sick and lose weight. Pregnant sows will abort when infected with PRV. Thus, an effective program for the eradication of PRV in swine is urgently needed.

Herpesviruses are among the most complex of animal viruses, coding for the synthesis of at least 50 virus specific proteins. Among the most immunologically reactive proteins of herpesviruses are the glycoproteins found in virion membranes and in the membranes of infected cells. The literature on pseudorabies glycoproteins (Ben-Porat, T., and Kaplan, A.S. (1970) Virology 41, 265-273; Kaplan, A.S., and Ben-Porat, T. (1970) Proc. Nat'l. Acad. Sci. USA 66, 799-806) indicates that there are at least four viral glycoproteins in infected cells and virions.

Glycoproteins excreted into the medium have been reported for several herpesviruses, including herpes simplex virus (Kaplan, A.S., Erickson, J.S., and Ben-Porat, T. (1975) Prog. Med, Virol. 21, 1-12); herpes saimiri virus (Randall, R.E., and Honess, R.W. (1980) J. Gen. Virol. 51, 445-449); Marek's disease virus (VanZaane, D., Brinkhof, J.M., Westenbrink, F., and Gielkens, A.L. (1981) Virology 121, 116-132); and pseudorabies virus (Erickson, J.S., and Kaplan, A.S. (1973) Virology 55, 94-102). In each of these cases except PRV, the excreted glycoprotein(s) is reported to be a subset of the virion glycoproteins. The publication by Ben-Porat and Kaplan, 1970, discloses that the PRV excreted glycoprotein is distinct from virion glycoproteins.

The excreted glycoprotein used in the subject invention is referred to herein as gX. This glycoprotein is known as 3a in the prior art. See Erickson and Kaplan, 1973, supr. Glycoprotein gX has the following characteristics when harvested from PRV-infected cells:

(1) it is the predominant protein in the culture medium of PRV infected animal cells in culture;

(2)  it is a glycoprotein;

(3)  it has a molecular weight of about 95 kilodaltons by standard SDS polyacrylamide gel electrophoresis (DATD crosslinked gel);

(4)  it is a sulfated protein;

(5)  it is soluble in about 1% perchloric acid;

(6)  it is immunogenic in standard laboratory mice; and,

(7)  it raises an immune response in the animal host which response is protective against a lethal challenge by a virulent strain of PRV.

## BRIEF SUMMARY OF THE INVENTION

The subject invention concerns the novel use of glycoprotein gX to protect PRV-susceptible animal hosts.  The use of gX in such a manner is unexpected in view of the prior art which heretofore recognizes only virion proteins as capable of raising a protective immune response in a susceptible animal host.  The prior art, as discussed above, does not recognize gX as a virion protein. The use of gX, or immunogenic fragments thereof, to treat animals can be accomplished in the form of various vaccine formulations disclosed herein and known in the art.

PRV-susceptible hosts are most mammals.  From an economic standpoint, swine are perhaps the most important.  Other hosts are ruminants, for example, cattle, sheep and goats, as well as companion and other animals, for example, dogs, cats, and the like.

## DETAILED DISCLOSURE OF THE INVENTION

Glycoprotein gX can be prepared by methods disclosed herein and by methods disclosed in the art.  See Erickson, J.S., Virology, 71, 598-601 (1976).  As noted above, gX is known in the prior art as 3a. The characteristics of gX are as disclosed above.

Methods are well known which can produce proteins or peptides antigenically equivalent to gX or fragments of gX.  Such methods include use of recombinant DNA to produce gX or fragments of gX in bacteria, yeast, or mammalian cells; or chemical synthesis of antigenic peptides.  This invention includes use of gX or fragments of gX, regardless of source, as a PRV vaccine.

The subject invention includes the use of gX in combination with other immunizing agents, with and without antibiotics, to treat PRV-susceptible animal hosts. Such other immunizing agents may be, for example, agents against infectious bovine rhinotracheitis, parainfluenza, Haemophilus somnus, Pasteurella haemolytica and multocida bacterin, and the like. Glycoprotein gX also can be used with other PRV viral proteins to protect PRV-susceptible animal hosts. Further, it is contemplated that synthetic peptide part(s) of gX can be used in the same manner as gX, as described herein.

The following disclosure is illustrative of the products and process of the invention, but is not to be construed as limiting. All percentages are by weight and all solvent mixture proportions are by volume unless otherwise noted.

## MATERIALS AND METHODS

### Cells and Virus

Cells used for virus propagation and protein production are African Green Monkey (C. aethiops) kidney cells (ATCC CCL81; =VERO). Cells are maintained in Dulbecco's modified Eagle's medium (GIBCO, Grand Island, N.Y.) supplemented with 100 units/ml penicillin, 100 $\mu$g/ml streptomycin, 10 mM HEPES (pH 7.4), 2 mM glutamine, and 10% fetal bovine serum (KC Biological).

Pseudorabies virus, is isolated from a natural outbreak of PRV in swine. Virus stocks are prepared by infecting either Vero or rabbit kidney cells at low multiplicity ($< 0.01$).

### Purification of Glycoprotein gX

PRV glycoprotein gX is purified from tissue culture supernatants of cells late in infection. Purification is facilitated by use of serum free medium to reduce the amount of contaminating protein. Vero cells grownin 850 cm2 roller bottles are infected with 5-10 plaque forming units per cell in medium 199 plus 1% fetal bovine serum. At 10-12 hours post-infection, the medium is carefully removed and the cells are rinsed gently with Dulbecco's modified Eagle's medium without any serum supplement. Thirty milliliters of this serum free medium is then added to the roller bottles followed by 4 hour incubation at 37°C. The supernatant is collected and clarified by centrifugation (5 min, 500 xg, room temperature) to remove floating cells and other gross debris. This medium is clarified further by ultracentrifugation over a 10% sucrose/20 mM Tris HCl (pH 7.4) cushion (125,000 xg, SW28 rotor, 4°C, 3 hrs) to remove cellular debris. The supernatant from this clarification is brought to 1% final concentration perchloric acid and incubated 10 minutes on ice. The perchloric acid precipitable material is removed by centrifugation (10 min, 16,000 xg, 4°C). The pellet is discarded and protein in the supernatant is neutralized with Tris base and precipitated with 10 volumes cold acetone incubated 12 hours at -20°C. Glycoprotein gX is precipitated with several other proteins under these conditions and is collected by centrifugation conditions identical to those for collecting the perchloric acid precipitate. The pelleted material is dried in vacuo.

Electrophoresis

SDS polyacrylamide gel electrophoresis is in 9.75% polyacrylamide gels with DATD crosslinker, as described by Morse, L., Pereira, L., Roizman, B., and Schaffer, P.A. J. Virol. 26, 389-410 (1978).

Animals

For the immunogenicity experiment, standard female CBA mice (age 4-6 weeks) are used. For the studies involving challenge with virulent PRV, standard CF1 adult mice are used. Twenty mice are used for a PRV titration to determine the optimal inoculum size as outlined in Table 2 (Study 1). The remaining mice are used to determine the effect of the gX preparation on protecting mice from PRV infection as outlined in Table 3 (Study 2). They are fed a commercial mouse chow. Preparation comprises essentially gX as the major viral component.

Immunizations

The protein extract from one 850 cm² roller bottle of infected cells is resuspended in 1 ml normal saline. For the first immunization (day 1), the suspension is mixed with an equal volume of complete Freund's adjuvant (Difco). In the immunogenicity experiment, the first immunization consists of 0.3 ml emulsion subcutaneously (SC) in the groin; in the protection experiment, the immunization is given in two sites, 0.1 ml intraperitoneally (IP) and 0.1 ml SC. The second immunization is on day 20 for the antigenicity experiment, and day 22 for the protection experiment. The second dose of antigen is given in normal saline, 0.2 ml SC for the immunogenicity experiment, and 0.1 ml each IP and SC for the protection experiment. Animals in the immunogenicity experiment are bled from the tail on day 27 for serum anti-PRV antibodies. Animals in the protection experiment are given the virus challenge on day 29.

ELISA for Anti-PRV Antibodies

3903
0115442

Virus infected cells are prepared by infecting confluent 150 cm2 flasks of Vero cells with 0.1-1 plaque forming units of PRV-Rice per cell. Infected cells are harvested at 16-20 hours post infection by shaking the flasks and centrifugation of the cells from the medium. The cells are washed three times in phosphate buffered saline by centrifugation.

Antibodies and reagents used in the ELISA assay are obtained from New England Nuclear unless otherwise indicated. The method described here is a modification of that of Kennett, R.H., Monoclonal Antibodies: Hybridomas, A New Dimension in Biological Analyses, Plenum Press, NY, pp. 376-377 (1980). The steps of the ELISA assay are as follows:

1. Poly-L-lysine at 0.01 mg/ml is added (0.05 ml) to microtiter wells for 30 minutes at 37°C to act as a support matrix. Following the incubation period the plates are flicked to remove excess poly-L lysine.

2. Virus infected cells (0.1 ml) are added to the wells. The equivalent of a confluent 150 cm2 flask is added to each 96 well microtiter plate. The plates are centrifuged (400 x g, in a Beckman TJ-6 centrifuge with microtiter plate carriers) tolayer the cells onto the flat microtiter wells.

3. Next 0.1 ml of glutaraldehyde (0.5% in PBS) is added for 30 minutes at room temperature to fix the cells. The plates are washed 3 times with 0.2 ml/well of PBS.

4. Unreacted sites for nonspecific binding of material to the polystyrene are blocked using 10% fetal calf serum (FCS) containing PBS for 30-60 minutes at room temperature. The plates are then washed once with PBS (0.2 ml/well).

5. In the first incubation 0.05 ml of test sample (i.e., dilutions of mouse serum samples) is added to duplicate wells and incubated for 16-18 hours at 4°C. The plates are then washed 3 times with PBS (0.2 ml/well).

6. In the second incubation 0.1 ml of horseradish peroxidase conjugated sheep F(ab')$_2$ anti-mouse immunoglobulin antibody made up in PBS pH 7.4 with 1% FCS, is added to the wells for 90-120 minutes at 37°C. The plates are then washed 3 times with PBS (0.2 ml/well).

7.  The final incubation consists of the addition of substrate (0.1 ml) specific for horseradish peroxidase. O-phenylenediamine (Sigma) is made up at 1 mg/ml in 17 mM citric acid 65 mM phosphate with 0.02% $H_2O_2$ pH 6.3. Conversion of enzyme substrate as indicated by a yellow-brownish color is apparent at 10-60 minutes and optical density values are determined using a spectrophotometer set at 450 nm (Titer-TeK microtiter plate reader). The enzymatic reaction is usually stopped with the addition of 0.05 ml per well of 0.5 N $H_2SO_4$.

Immunogenicity of gX Preparation

The gX preparation is injected into mice and the sera of these mice is analyzed by an ELISA assay designed to detect mouse antibody reactive with PRV-infected cells. As can be seen in Table 1, a clear quantitative difference can be found in the anti-PRV concentration for animals receiving preparations from mock- or PRV-infected cells. Those animals which receive the mock preparation of the excreted glycoprotein react slightly above that of normal mouse sera. This reactivity reflects the presence of antibodies to Vero cell-associated antigens as confirmed in a second assay using rabbit kidney cells in which the mock-immunized mouse serum react essentially like normal mouse serum. Those animals which receive the gX preparation show much higher levels of antibody reactive with PRV-infected Vero cells. The sera from a hyperimmunized donor give the highest titer of reactive antibody reflecting the immunogenicity of wild type virus after repeated immunizations.

Protective Immunization of Mice with gX Preparation

The gX preparation is tested for its ability to elicit an immune response in mice that would give protection from a lethal dose of virulent PRV. The results of two studies are presented. Study 1 is a preliminary experiment to determine the appropriate challenge dose of PRV-Rice in mice. Study 2 is a blind comparison of the protective effect of crude protein preparations from uninfected cells (designated mock preparation) or PRV-infected cells (designated gX).

Study 1

Mice were allotted randomly to 5 groups of 4 mice each. Each group is kept in a separate cage. Mice are inoculated IP with 0.1 ml of the viral inoculum. They are observed daily and mortality is recorded.

The results obtained from the viral titration are listed in Table 2. All mice inoculated with $10^4$ PFU died within 3 to 4 days with a mean day of death (MDD) of 3.0 to 3.75. Three of 4 mice inoculated with $10^3$ PFU/ml died with a MDD of 4.67. The challenge dose for Study 2 is $10^4$ PFU/ml.

Study 2

Three groups of mice are used to determine whether or not the gX preparation is capable of protecting mice against PRV infection. Group 1 contains 8 mice treated twice with the gX preparation. Group 2 contains 9 mice treated twice with the mock preparation. Group 3 contains 8 mice as non-treated Control A. In addition, 4 more mice are added as non-treated Control B. Groups 1 and 2 mice are treated initially with the adjuvant suspensions of gX or mock and three weeks later they are given a booster injection of the saline suspension. One week after the last injection, all the mice are challenged IP (0.1 ml) with PRV ($10^4$ PFU/ml). Mice are observed daily for mortality and the study is terminated 8 days after viral challenge.

The data obtained from the effect of gX on PRV infection in mice are shown in Table 4. Mortality is recorded in 6 of 8 (75%) non-treated Control A mice, 4 of 4 (100%) non-treated Control B mice, 8 of 9 (88.9%) mock treated mice and 1 of 8 (12.5%) gX treated mice. The MDD/group is 3.0, 3.6, 4.0 and 5.0, respectively. These data indicate that the gX given to mice is capable of protection against PRV infection as reflected by a reduction in rate of mortality. Figure 4 summarizes the mortality data from this study.

## TABLE 1

### SERUM SOURCE

|  | Hyper-immunized Mouse | Normal Mouse | Mock, Mouse 1 | Mock, Mouse 2 | gX, Mouse 1 | gX, Mouse 2 |
|---|---|---|---|---|---|---|
| 1/50 | .411 | .185 | .172 | .225 | .326 | .270 |
| 1/200 | .450 | .123 | .150 | .193 | .267 | .276 |
| 1/800 | .355 | .048 | .068 | .073 | .157 | .124 |
| 1/3200 | .189 | .006 | .031 | .010 | .127 | .038 |

Data summary of ELISA assay for anti-PRV antibodies in immunized mice. The ELISA assay is performed as described above. Numbers in the table represent optical density readings, which measure the amount of horseradish peroxidase substrate converted and therefore anti-PRV antibody concentration. The numbers represent an average of duplicate assays. The hyperimmunized mouse is immunized with a less virulent, iododeoxyuridine-resistant mutant PRV strain, then given repeated challenges with virulent PRV strains. Mock or gX animals receive the protein preparation from uninfected or PRV infected Vero cells, respectively.

## TABLE 2

Mortality in mice following intraperitoneal (IP) inoculation (0.1 ml/mouse) with various inoculum sizes of pseudorabies virus (Rice Strain).

| No. Mice | Inoculum Size/ml Plaque Forming Unit | No. Mortality | Mean Day Death |
|---|---|---|---|
| 4 | 106 | 4 | 3.00 |
| 4 | 105 | 4 | 3.75 |
| 4 | 104 | 4 | 3.75 |
| 4 | 103 | 3 | 4.67 |
| 4* | None | 0 | ---- |

*Used later in the challenge study as nontreated B (see Tables 2 and 3).

## TABLE 3

Experimental design to determine the effect of a gX preparation on protecting mice against PRV infection.

| Group No. | Group Status | No. Mice Used | Treatment Day Day 1 | Day 22 | Viral Challenge Day 29 |
|---|---|---|---|---|---|
| 1 | Glycoprotein | 8 | Adj. Susp.[a] | Saline[b] | 0.1 ml IP[c] |
| 2 | Mock | 9 | Adj. Susp.[a] | Saline[b] | 0.1 ml IP |
| 3 | Nontreated Control A | 8 | None | None | 0.1 ml IP |
| 4 | Nontreated Control B | 4 | None | None | 0.1 ml IP |

[a]The complete adjuvant suspension is injected 0.1 ml intraperitoneally (IP) and 0.1 ml subcutaneously.

$^b$The saline suspension is injected 0.1 ml intramuscularly and 0.1 ml subcutaneously.

$^c$The PRV is inoculated 0.1 ml IP.

TABLE 4

Effect of gX in protecting mice against pseudorabies viral infection.

| Group No. | Group Status | No. Mice Used | Mortality No. | % | Mean Day of Death |
|---|---|---|---|---|---|
| 1 | Glycoprotein X | 8 | 1 | 12.5 | 5.0 |
| 2 | Mock | 9 | 8 | 88.9 | 4.0 |
| 3 | Nontreated Control A | 8 | 6 | 75.0 | 3.6 |
| 4 | Nontreated Control B | 4 | 4 | 100.0 | 3.0 |

The immunogen gX prepared in accordance with the present invention is preferably free of PRV virus. Thus, the vaccine immunogen of the subject invention is composed substantially entirely of the desired immunogenic protein.

The immunogen can be prepared in vaccine dose form by well-known procedures. For parental administration, such as intramuscular injection, the immunogen may be combined with a suitable adjuvant, for example, aluminum hydroxide. Such adjuvants are available commercially from various sources, for example, Merck Adjuvant 65 (Merck and Company, Inc., Rahway, N.J.). Another suitable adjuvant is Freund's Incomplete Adjuvant (Difco Laboratories, Detroit, Michigan). The proportions of immunogen and adjuvant can be varied over a broad range so long as both are present in effective amounts. For example, aluminum hydroxide can be present in an amount of about 0.5% of the vaccine mixture ($Al_2O_3$ basis). On a per dose basis, the concentration of the immunogen can range from less than 1.0 mg to about 4.0 mg. A preferable range is from about 1.0 to about 3.0 mg per dose. A suitable dose size is about one milliliter. Accordingly, a dose for intramuscular injection would comprise 1 ml containing 1.0 mg of immunogen in admixture with 0.5% aluminum hydroxide. Comparable dose forms can also be prepared for parenteral administration to baby pigs, but the amount of immunogen per dose will be smaller, for example, about 0.25 to about 1.0 mg per dose. The immunogen can also be prepared in the form of an enteric-coated oral vaccine, for example, as described in U.S. Patent 3,823,228.

For vaccination of sows, advantageously, a 2-dose regimen can be used. The first dose can be given from about several months to about 5 to 7 weeks prior to farrowing. For example, the sows can be vaccinated in the fall, such as about the first week in November. The second dose of the vaccine then should be administered several weeks after the first dose, for example, about 2 to 4 weeks later and vaccine can then be administered up to but prior to farrowing. Alternatively, the immunogen can be given as a single 2-ml dose, for example, at about 5 to 7 weeks prior to farrowing. However, a 2-dose regimen, described above, is considered preferable for the most effective immunization of the baby pigs. Semi-annual revaccination is recommended for breeding animals. Boars may be revaccinated at any time. Also, sows can be revaccinated before breeding. All vaccine administrations can be done by intramuscular injection.

CLAIMS

1.    A vaccine for protecting an animal susceptible to pseudorabies virus (PRV) infection, which comprises a material named gX, or an immunogenic fragment thereof, gX being characterised as follows:

    (1)   it is a glycoprotein;

    (2)   it has a molecular weight of about 95 kilodaltons by standard SDS polyacrylamide gel electrophoresis (DATD cross-linked gel);

    (3)   it is a sulfated protein;

    (4)   it is soluble in 1% perchloric acid;

    (5)   it is immunogenic in standard laboratory mice; and

    (6)   it raises in the animal an immune response which is protective against a lethal challenge by a virulent strain of PRV.

2.    A vaccine for protecting an animal, which comprises the material gX as characterised in claim 1, or an immunogenic fragment thereof, in combination with one or more other immunising agents.

3.    A vaccine according to claim 1 or claim 2, which comprises a PRV-viral protein in addition to the material gX.

4.    A vaccine according to any preceding claim, wherein the animal is a pig.